# EUROPEAN PATENT SPECIFICATION

(11) **EP 2 732 833 B1**
(45) Date of publication and mention of the grant of the patent: **29.07.2015**
(21) Application number: 12192584.6
(22) Date of filing: 14.11.2012
(51) Int. Cl.: A61M 1/00, F16K 31/60, F16K 35/02, G05D 16/00, G05D 7/00

(54) **Regulator for fluid suction and / or supply systems for medical use**
Regulator für Fluidansaug- und / oder Fluiversorgungssysteme für medizinische Anwendungen
Régulateur pour des systèmes d'aspiration et / ou d'alimentation de fluide pour usages médicaux

(43) Date of publication of application: 21.05.2014
(73) Proprietor: Flow Meter S.p.a., 24040 Levate (Bergamo) (IT)
(72) Inventor: Paratico, Roberto, 24040 Levate (Bergamo) (IT)
(74) Representative: Botti, Mario

(56) References cited:
- EP-A1- 1 757 317
- EP-A1- 2 075 017
- EP-A1- 2 151 731
- DE-A1-102009 025 441
- DE-U1-202006 018 973
- US-A1- 2009 039 304

## Description

### Field of application

The present invention relates to a regulator unit for fluid suction and/or supply systems for medical use, in particular used in a medical aspiration system or in oxygen therapy.

The regulator unit may be in particular a vacuum regulator, an aspirator or a flowmeter.

### Prior Art

In the medical sector there exist various fluid suction and supply systems which use internally components and units intended to measure and regulate operating parameters of the system itself.

These systems comprise, for example, systems for removing by aspiration the body liquids of a patient, which are controlled by a vacuum regulator arranged between the vacuum source and the storage vessel; or systems for supplying medical gas for oxygen therapy, where a flowmeter with flowrate reading and regulating functions is associated with the outlet of the medical-gas distribution network.

The regulator units of the type mentioned above generally have a regulating valve designed to regulate a flow path inside the device.

Thus, for example, in the vacuum regulator the valve intercepts an internal suction path so as to regulate the value of the vacuum.

Similar regulation may be obtained in a Venturi type suction device where the valve is used to throttle the compressed-air flow which produces the Venturi effect.

In a flowmeter, the regulating valve will be designed to intercept the flow supply path, thus allowing regulation of the flowrate.

In some cases, as for example in the case of a flowrate regulator with calibrated orifices, the regulation performed by the device is of the discrete type, namely the operating parameter may assume only certain predetermined values. The control knob must therefore be locked only in certain angular positions.

In the devices of this type, means for performing snap-engaging locking of the knob are provided, said means consisting of interference balls which are pressed by a compressed spring, allowing the control member to be kept fixed in its operating position.

These measures, however, may not be adopted where the regulation permitted is of the continuous type, and the regulation knob is consequently free to assume any angular position with respect to the main body of the device.

In the devices of this latter type there exists therefore a drawback which hitherto has not been overcome, associated with the mobility of the said knob.

It may happen in fact that the regulation knob may suffer an accidental knock and be moved from its operating position, thus obliging the healthcare assistant to perform regulation again.

Valve devices, not pertaining to the field of the invention, are disclosed in prior art documents DE 10 2009 025441 A1 and US 2009/039304 A1.

A valve for a medical suction apparatus, provided with a regulation knob, is disclosed in prior art document DE 20 2006 018973 U1.

A suction assembly device, which is not provided with means for preventing accidental rotation of the regulating knob, is disclosed in EP 2 075 017 A1.

A Venturi vacuum system, which is not provided with means for preventing accidental rotation of the regulating knob, is disclosed in EP 1 757 317 A1.

The technical problem forming the basis of the present invention is therefore that of devising a regulator unit which has structural and functional characteristics such as to overcome the drawback mentioned above in connection with the prior art.

### Summary of the invention

The invention is defined by the features of independent claim 1.

The aforementioned technical problem is solved by a regulator unit for fluid suction and/or supply systems for medical use, comprising a main body provided internally with at least one regulating valve - for example a needle valve - which performs continuous regulation of a parameter of said fluid suction and/or supply system; and a regulation knob which can be accessed from the outside of the main body and is designed to control said regulating valve; the regulation knob is movable linearly along its axis between a regulating configuration, where it is rotationally movable with respect to the main body and controls the regulating valve, and a locking configuration, where it is rotationally locked with respect to the main body.

The possibility of setting the regulation knob to the locked position thus eliminates any risk of accidental rotation of this regulating member, without, however, limiting the continuous regulation of the device.

The regulation knob may, in particular, have a cup-like configuration and be fitted on top of a cylindrical collar integral with the main body. An internal surface of said regulation knob and an external surface of said cylindrical collar may have locking means and counter-locking means, respectively, which engage with each other when said regulation knob is in the locked configuration.

Said locking means and counter-locking means may advantageously consist of two sets of straight-teeth toothing which are respectively provided on an external circumference of the cylindrical collar and on an internal circumference of the regulation knob.

The two sets of straight-teeth toothing extending along the entire circumferential extension advantageously form a stable coupling between cylindrical collar and knob, irrespective of the specific angular position of the latter.

Said cylindrical collar has said toothing preferably arranged on its free end, such that the two sets of toothing may be easily disengaged by moving the regulation knob away from said cylindrical collar.

Said internal surface of the regulation knob and said external surface of said cylindrical collar may also have ridges cooperating together so as to keep said regulation knob alternately in the locked configuration or in the released configuration.

These ridges may comprise in particular a first radial projection and a second radial projection on one of the two surfaces, preferably the external surface of the cylindrical collar, and a circumferential ring embossed on the other surface, preferably on the inner rim of the regulation knob.

The circumferential ring may thus be constrained between the first and the second radial projections when the regulation knob is in the released configuration; said circumferential ring being constrained beyond said second radial projection when the regulation knob is in the locked configuration.

It should be noted that a further constraint on the knob, due to contact of the latter against a shoulder of the main body, allows the knob to be constrained in the locked configuration in the opposite direction to the direction of contact with the second radial projection. The regulation knob in the locked configuration in fact bears against said shoulder.

The second radial projection is advantageously connected by means of ramps to the surface of the cylindrical collar or the regulation knob, so as to allow the linear displacement of the knob between the two - locked and released - configurations. During displacement, in fact, the circumferential ring, sliding along the ramp and being deformed elastically, passes over the first radial projection, passing from one configuration to the other.

Said regulation knob may moreover comprise an internal bush which is designed to be slidably inserted inside the cylindrical collar, so as to guide the linear movement of the regulation knob and provide the locking/release mechanism with robustness and stability.

The regulation knob may have, on its external surface, a plurality of sectors which are divided up by a corresponding number of raised ribs.

This configuration, which advantageously improves the grip of said knob, may be advantageously obtained by producing the knob by means of co-injection moulding of two different plastic materials.

The regulator unit according to the present invention may be in particular a vacuum regulator, suitable for being connected to a vacuum source for the suction of fluids inside a safety storage vessel or other container.

In this case, the regulating valve intercepts a suction flow path inside the main body so as to allow the regulation of the system suction value.

The regulation knob may thus be rotationally associated with a cover of the main body, said cover being fixed to the rest of the main body by means of a bayonet mount.

Said bayonet mount allows advantageously rapid assembly and disassembly of the cover on the main body, for example performing a rotation through a quarter of a turn.

The regulator unit according to the present invention may be a vacuum generating unit comprising inside the main body a compressed air Venturi aspirator.

In this case, the regulating valve intercepts the compressed air flow of the Venturi aspirator o as to allow the regulation of the system suction value.

The regulator unit according to the present invention may also be a flowmeter, comprising an inlet designed to be connected to a medical gas distribution source and an outlet for supplying said medical gas to a user apparatus.

The regulating valve intercepts in this case a flow supply path between said inlet and said outlet so as to allow the regulation of the flowrate of the medical gas supplied at the outlet.

Further characteristic features and advantages of the regulator unit according to the invention will emerge from the description provided hereinbelow, of a number of examples of embodiment, provided by way of a non-limiting example, with reference to the accompanying drawings.

### Brief description of the drawings

Figure 1 shows a perspective view of a vacuum regulator according to the present invention;
Figure 2 shows a perspective view of the vacuum regulator according to

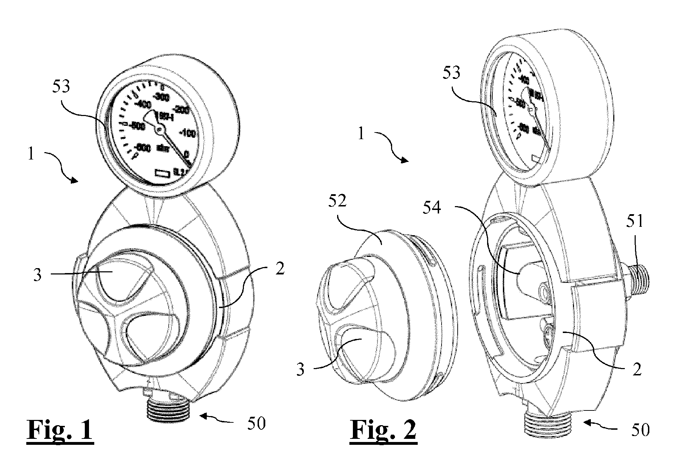

Figure 1 with the closing cover of the main body removed;

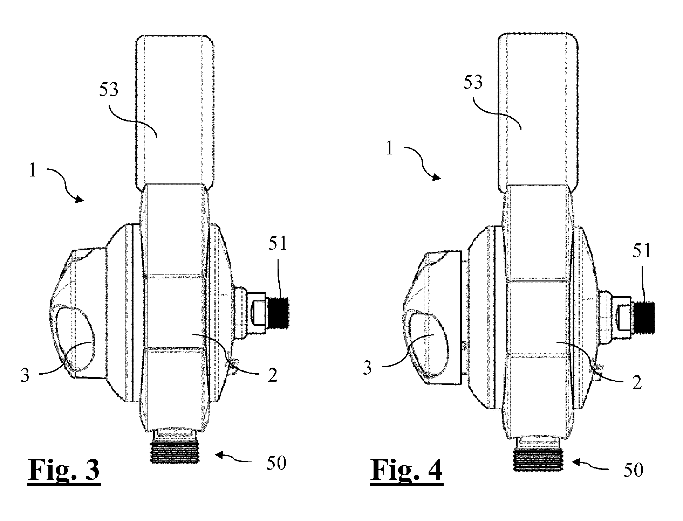

Figure 3 shows a side view of the vacuum regulator according to Figure 1, with the regulation knob in the locked configuration;
Figure 4 shows a side view of the vacuum regulator according to Figure 1, with the regulation knob in the released configuration;

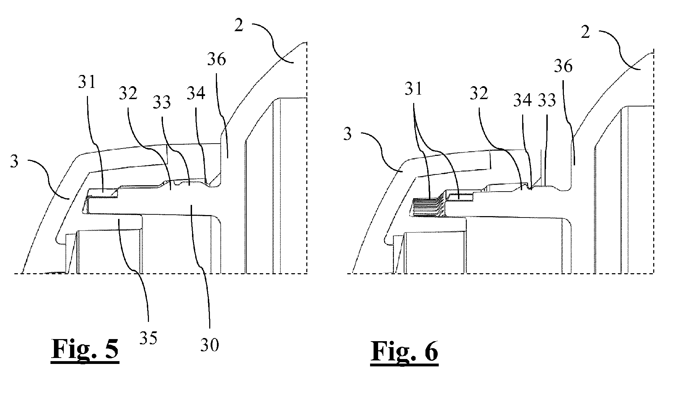

Figure 5 shows a view, cross-sectioned along a middle plane, of a detail of the vacuum regulator according to Figure 1, with the regulation knob in the locked configuration;
Figure 6 shows a view, cross-sectioned along a middle plane, of a detail of the vacuum regulator according to Figure 1, with the regulation knob in the released configuration;

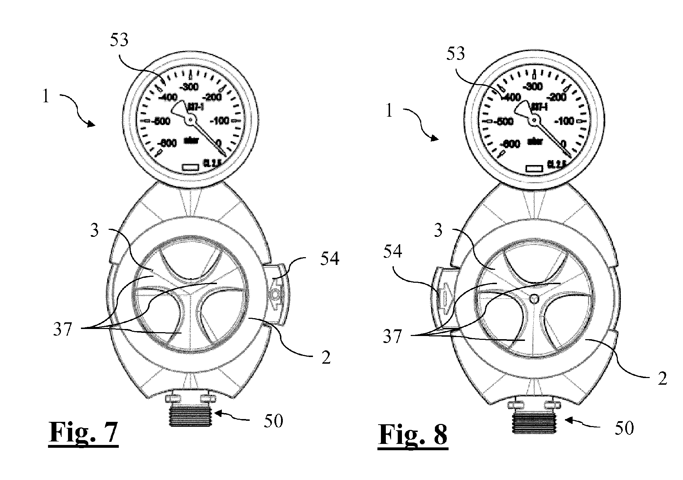

Figure 7 shows a front view of the vacuum regulator according to Figure 1, with the slide valve in the open configuration;
Figure 8 shows a front view of the vacuum regulator according to Figure 1, with the slide valve in the closed configuration;

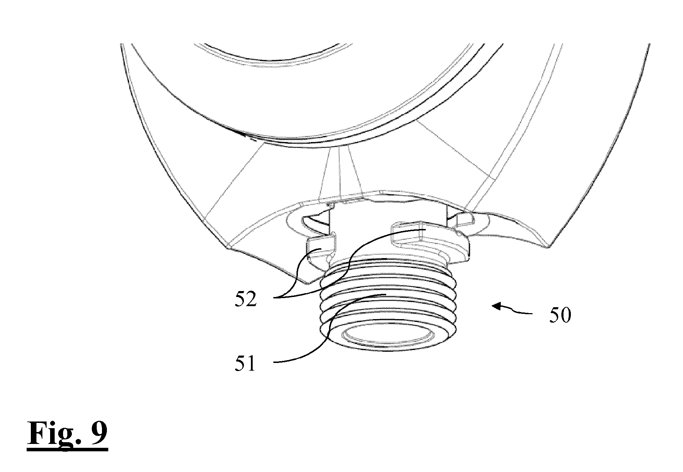

Figure 9 shows a perspective view of a detail of the vacuum regulator according to Figure 1;

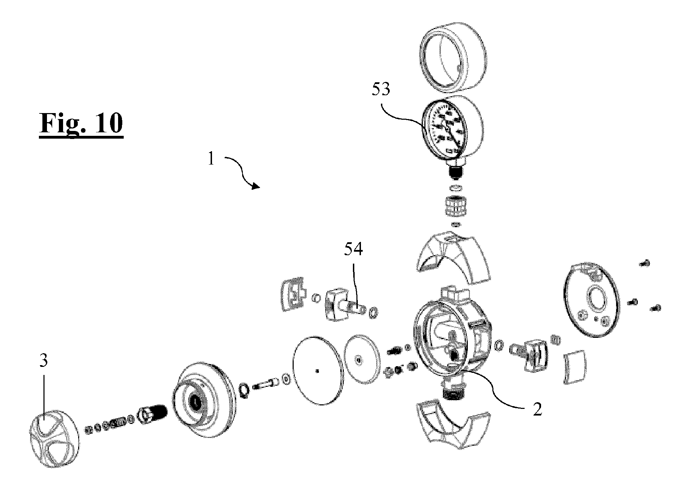

Figure 10 shows an exploded perspective view of the vacuum regulator according to Figure 1;

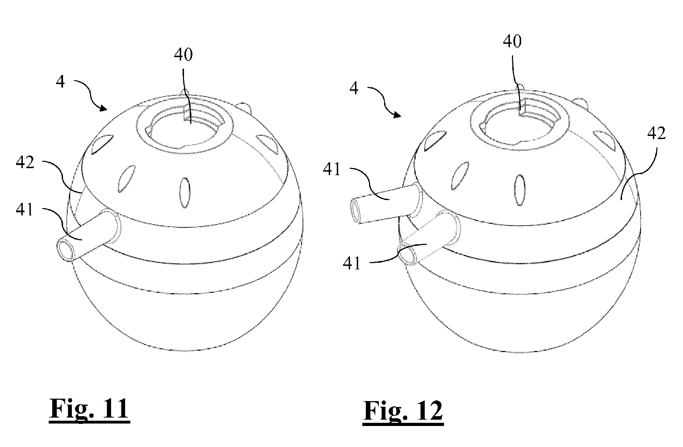

Figure 11 shows a perspective view of a safety storage vessel which can be associated with the vacuum regulator according to Figure 1;
Figure 12 shows another perspective view of the safety storage vessel according to Figure 11;

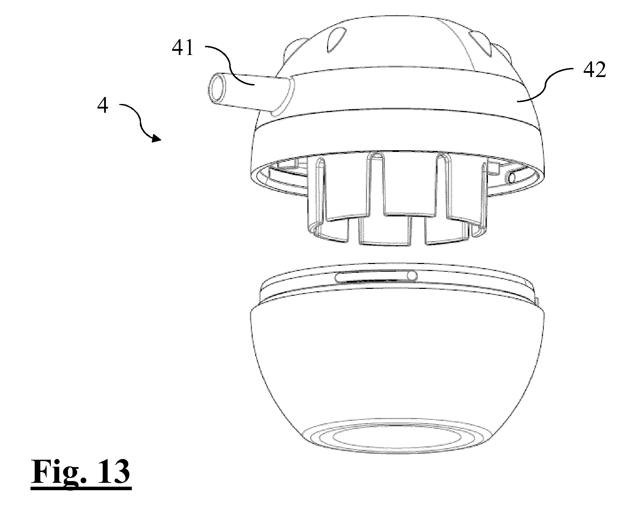

Figure 13 shows a perspective view of the safety storage vessel according to Figure 11 in an open configuration;

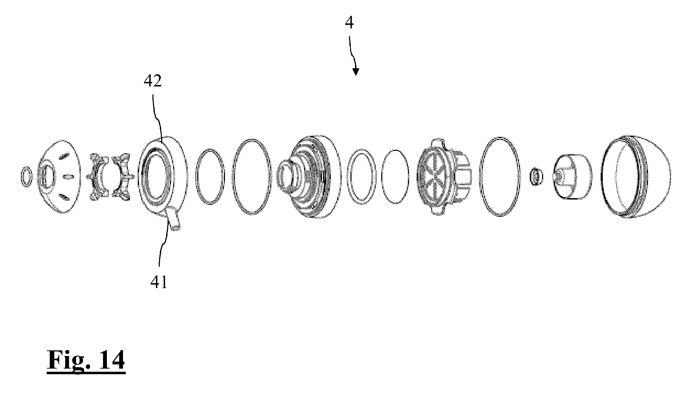

Figure 14 shows an exploded perspective view of the safety storage vessel according to Figure 11;

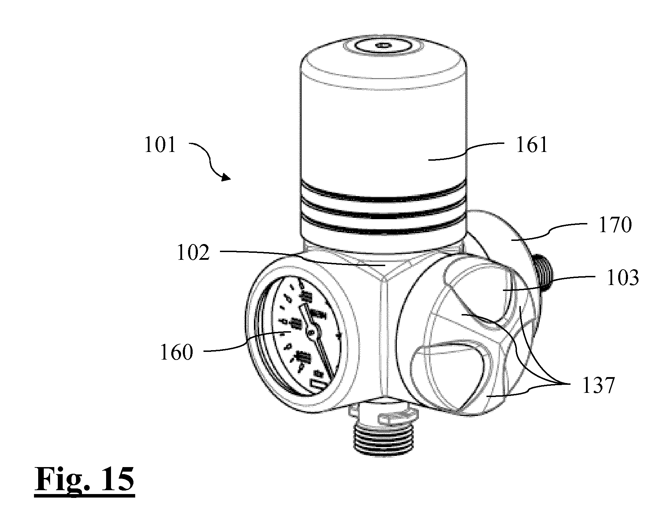

Figure 15 shows a perspective view of a vacuum generating unit according to the present invention;

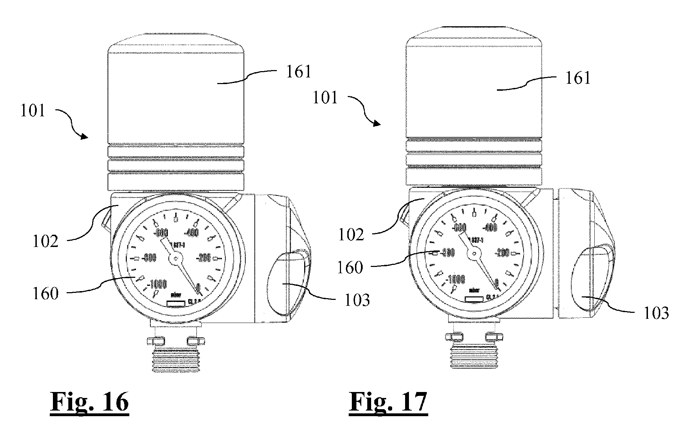

Figure 16 shows a side view of the vacuum generating unit according to
Figure 15, with the regulation knob in the locked configuration;
Figure 17 shows a side view of the vacuum generating unit according to
Figure 15, with the regulation knob in the released configuration;

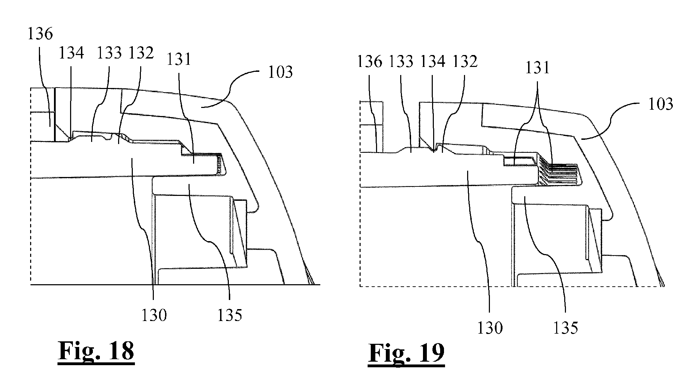

Figure 18 shows a view, cross-sectioned along a middle plane, of a detail of the vacuum generating unit according to Figure 15, with the regulation knob in the locked configuration;
Figure 19 shows a view, cross-sectioned along a middle plane, of a detail of the vacuum generating unit according to Figure 15, with the regulation knob in the released configuration;

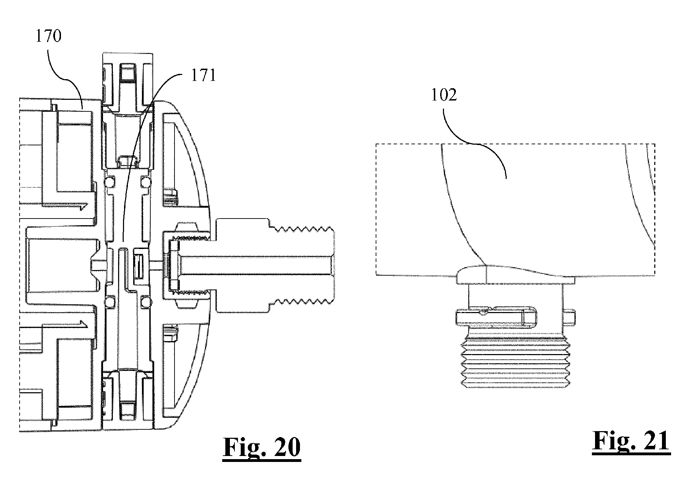

Figure 20 shows a view, cross-sectioned along a middle plane, of the slide valve of the vacuum generating unit according to Figure 15;
Figure 21 shows a perspective view of a detail of the vacuum generating unit according to Figure 15;

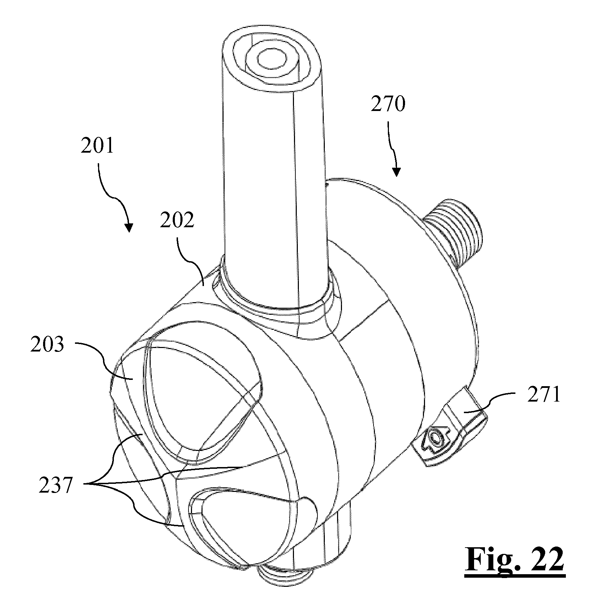

Figure 22 shows a perspective view of a flowmeter according to the present invention;

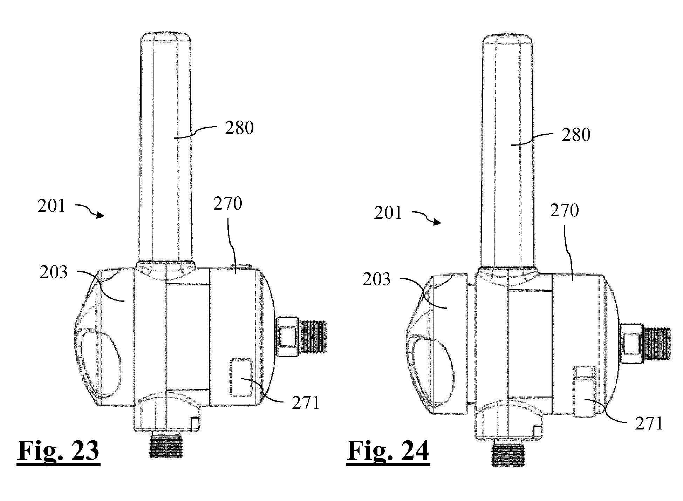

Figure 23 shows a side view of the flowmeter according to Figure 22, with the regulation knob in the locked configuration;
Figure 24 shows a side view of the flowmeter according to Figure 22, with the regulation knob in the released configuration;

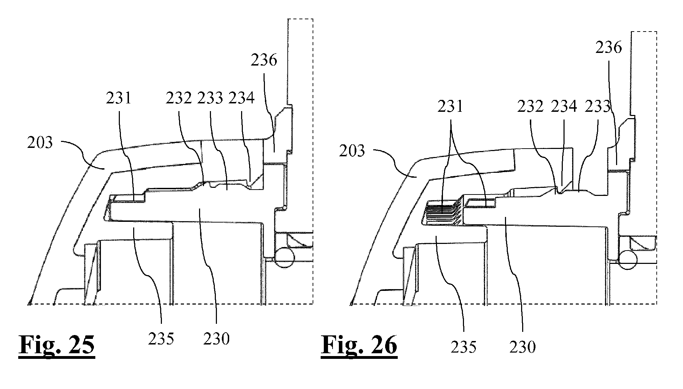

Figure 25 shows a view, cross-sectioned along a middle plane, of a detail of the flowmeter according to Figure 22, with the regulation knob in the locked configuration;
Figure 26 shows a view, cross-sectioned along a middle plane, of a detail of the flowmeter according to Figure 22, with the regulation knob in the released configuration;

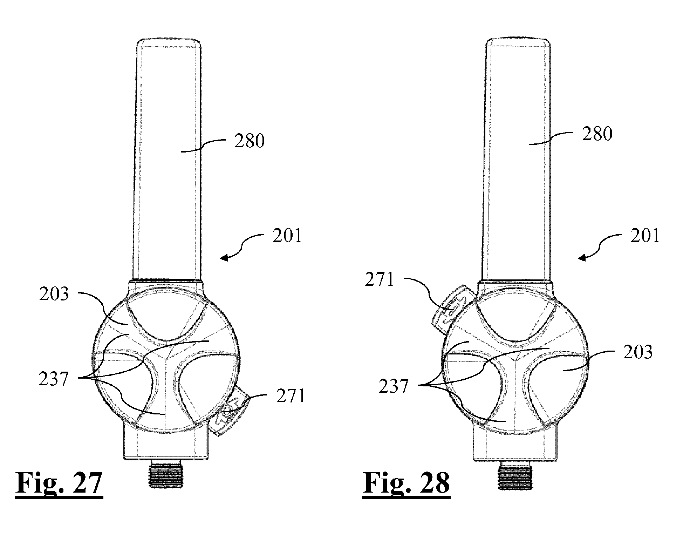

Figure 27 shows a front view of the flowmeter according to Figure 22, with the slide valve in the open configuration;
Figure 28 shows a front view of the flowmeter according to Figure 22, with the slide valve in the closed configuration;

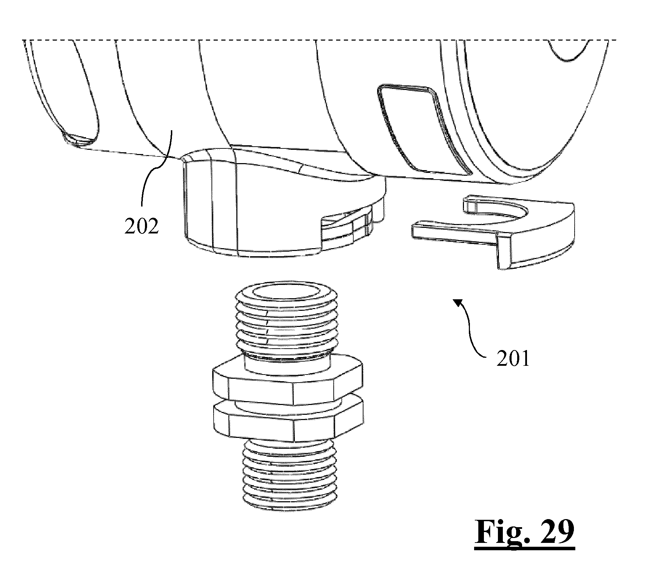

Figure 29 shows a perspective view of a detail of the flowmeter according to Figure 22;

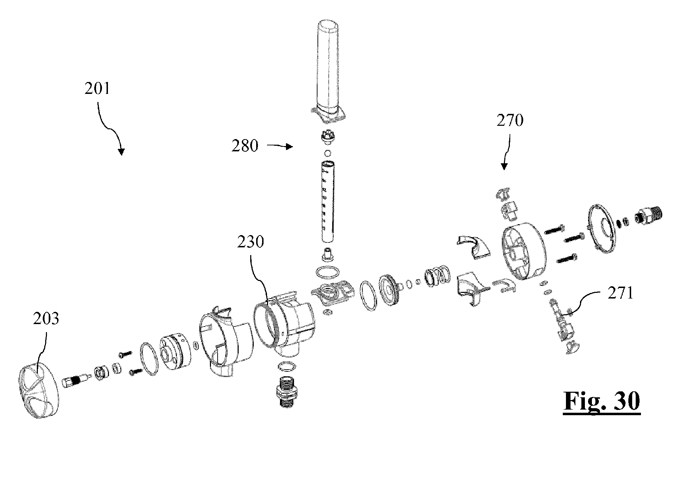

Figure 30 shows an exploded perspective view of the flowmeter according to Figure 22.

### Detailed description

With reference to the accompanying Figures 1 to 10, 1 denotes in general a first embodiment of the regulator unit according to the present invention, which in the case in question takes the form of a vacuum regulator.

The vacuum regulator 1 forms, in combination with a safety storage vessel 4 shown in Figures 11 to 14, a suction assembly suitable for being connected to a vacuum source for aspirating fluids, in particular organic liquids.

The vacuum regulator 1 has in fact a bottom connection 50 designed to be sealingly fixed inside a top inlet opening 40 of the safety storage vessel 4, or to a storage container of another type.

As can be noted in fact from the view, on a larger scale, shown in Figure 9, said bottom connection 50 comprises both a threaded connection 51, designed to be connected to storage containers different from the safety storage vessel 4, and two lugs 52 designed to engage, by means of a bayonet mount, inside the top inlet opening mentioned above.

The suction assembly defines internally a suction path, extending between at least a first nozzle 41 projecting laterally from the safety storage vessel 4 and suitable for connection, by means of a flexible pipe, to a primary storage container for the liquids to be removed, and a second nozzle 51 associated with the vacuum regulator 1 and suitable for being connected to the vacuum source.

The vacuum regulator 1 essentially takes the form of a continuous-regulating valve, for example a needle valve, arranged so as to intercept the suction flow path and allow regulation of the system suction value.

Said regulating valve is contained inside a main body 2 with a substantially cylindrical form and formed by a casing, an end wall and a cover 52.

The cover 52 is equipped with a regulation knob 3 which controls in a known manner said needle valve.

As can be noted in Figure 2, said cover 52 is fixed to the casing by means of bayonet mount, such that it is sufficient to rotate the cover by a quarter of a turn in order to perform the assembly and disassembly operations.

The vacuum regulator 1 is completed by a vacuum meter 53 which is connected in a known manner to the main body 2. Moreover, a shut-off slide valve 54 is arranged between the second nozzle 51 and the flow regulating valve and allows the suction flow path to be selectively interrupted.

It should be pointed out that regulation of the vacuum and measurement by means of the vacuum meter are performed in a known manner, which does not fall within the present invention, and therefore they will not be described in the present invention.

The safety storage vessel 4 also comprises internally components of the known type, such as an overflow valve and a filter, which will not be described further.

It should be noted, however, how the first nozzle 41 of the safety storage vessel 4, consisting of a rubber-holder nozzle, is advantageously formed as one piece with a rotatable annular portion 42 of the device body. Thus, an operator may easily modify the position of the first nozzle 41 according to specific needs, without having to disassemble the safety storage vessel from the vacuum regulator 1.

Moreover, the number of first nozzles 41 formed on the annular portion may be greater than one, for example equal to two, as shown in Figure 12.

The mechanism for locking/releasing the regulation knob 3 will instead now be considered, said regulation knob forming the main subject of the present invention.

Said regulation knob 3 is in fact movable linearly along its axis between a regulating configuration, where it is rotationally movable with respect to the main body 2 and controls the regulating valve, and a locking configuration, where it is rotationally locked with respect to the main body 2.

The main body 2 has, projecting from its front surface, a cylindrical collar 30 on top of which the knob 3 is engaged. At the base of said cylindrical collar 30 the front surface of the main body 2 defines a shoulder 36.

The regulation knob 3 has a cup-like configuration with an outer side wall which is fitted slidably over the cylindrical collar 30 mentioned above; it also has an internal bush 35 which is concentric with the outer side wall and designed to be introduced slidably inside the cylindrical collar 30, acting as a further guide for the linear sliding movement of the regulation knob.

The outer side wall of the regulation knob 3 has a circumferential ring 34 projecting from its inner rim. This circumferential ring 34 is thus able to cooperate with radial projections 32, 33 formed on the external surface of said cylindrical collar 30.

The second radial projection 33, which is arranged in the vicinity of the shoulder 36, is connected by means ramps to the surface of the cylindrical collar and has a height slightly lower than that of the first radial projection 32. Said second radial projection 33 can therefore by passed over by the circumferential ring 34. If a sufficient force is applied by the operator, the circumferential ring 34 is in fact able to be deformed elastically when moving over one of the ramps so as to pass from a first position, situated between shoulder 36 and second radial projection 33, into a second position, situated between first radial projection 32 and second radial projection 33, and vice versa. As will become clear from the description which follows, the aforementioned first position is equivalent to the locked configuration of the knob, while the second position is equivalent to the released or regulating position.

When, in fact, the circumferential ring 34 is in the first position, the cylindrical collar 30 is completely inserted inside the regulation knob 3. The outer rim of the cylindrical collar 30, as well as the base of the side wall of the regulation knob 3, are provided with sets of straight-teeth toothing 31 which engage with each other when the regulation knob 3 is in this position. Rotation of the knob with respect to the main body 1 is therefore not possible.

By pulling the regulation knob 3 outwards, it is however possible to move the circumferential ring 34 beyond the second projection 33, until it is locked against the first projection 32 in a released configuration of the regulation knob 3.

In this released configuration, the teeth 31 are not engaged with each other and it is thus possible to operate the regulation knob 3.

It should be noted that, in order to increase the grip of the regulation knob 3, it may be advantageously obtained by means of co-injection moulding from two different plastic materials. Three radial sectors, which are divided from each other by a corresponding number of raised ribs 37, are thus defined.

With reference to Figures 15 to 21, 101 denotes in general a second embodiment of the regulator unit according to the present invention, which in the case in question takes the form of a vacuum generating unit.

The vacuum generating unit 101 comprises in a known manner, inside the main body 102, a compressed air Venturi aspirator. It also comprises a regulating valve designed to intercept the compressed air flow of the Venturi aspirator so as to allow the regulation of the system suction value.

A regulation knob 103 is designed to control said regulating valve. Said regulation knob 103 has a locking mechanism which is entirely similar to the one described above with reference to the regulation knob 3 of the vacuum regulator 1. The similarity between the two mechanisms is evident from an analysis of the attached figures, where elements and parts, which are identical or similar to those of the first embodiment, are identified by adding 100 to the identification number previously used.

Other known elements of the vacuum generating unit 101 consist of the vacuum meter 160 and the silencer 161.

It should also be noted that the main body 102 has a stop flow stage 170 formed inside a first shell made by means of moulding as one piece.

Said first shell, with an essentially cylindrical outer form, is passed through transversely by a slide valve 171 which intercepts the flow supply path.

The slide valve 171 has a stem inside a diametral hole of the first shell 10. This stem is movable between a closing position, in which it blocks the flow supply path, and an open position, in which said flow supply path is not blocked.

Two oppositely arranged pushbuttons are integral with the ends of the stem and project alternately from the cylindrical surface of the main body 102 depending on whether the slide valve 171 is in the open configuration or in the closed configuration.

It should be noted that the diametral hole which receives the stem has an offset axis, preferably inclined at 45°, relative to the vertical middle plane. This orientation of the slide valve 30 allows the overall dimensions to be advantageously reduced.

With reference to Figures 22 to 30, 201 denotes in general a third embodiment of the regulator unit according to the present invention, which in the case in question takes the form of a flowmeter.

The flowmeter 201 comprises, in a known manner, inside a main body 202, a flow regulating stage and a variable-area flowrate measuring device 280. The flow measurement stage comprises in particular a regulating valve which is designed to intercept the flow of medical gas passing through the flowmeter.

A regulation knob 203 is designed to control said regulating valve. Said regulation knob 203 has a locking mechanism which is entirely similar to the one described above with reference to the regulation knob 3 of the vacuum regulator 1. The similarity between the two mechanisms is evident from an analysis of the attached figures, where elements and parts, which are identical or similar to those of the first embodiment, are identified by adding 200 to the identification number previously used.

It should also be noted that the flowmeter 201 envisages a stop flow stage 270 which is entirely similar to that described with reference to the vacuum generating unit 101. Once again, the similarity between the two stages is evident from an analysis of the attached figures, where elements and parts, which are identical or similar to those of the second embodiment, are identified by adding 100 to the identification number previously used.

Obviously, a person skilled in the art, in order to satisfy any specific requirements which arise, may make numerous modifications and variations to the regulator unit described above, all of which being moreover contained within the scope of protection of the invention, as defined by the following claims.

## Claims

1. Regulator unit (1; 101; 201) for fluid suction and/or supply systems for medical use, comprising a main body (2; 102; 202) provided internally with at least one regulating valve which performs continuous regulation of a parameter of said fluid suction and/or supply system; and a regulation knob (3; 103; 203) which can be accessed from the outside of the main body (2; 102; 202) and is designed to control said regulating valve; wherein said regulation knob (3; 103; 203) is movable linearly along an axis thereof between a regulating configuration, where it is rotationally movable with respect to the main body (2; 102; 202) and controls the regulating valve, and a locking configuration, where it is rotationally locked with respect to the main body (2; 102; 202); wherein said regulation knob (3; 103; 203) has a cup-like configuration; **characterized in that** said regulation knob (3; 103; 203) is fitted on top of a cylindrical collar (30; 130; 230) which is integral with the main body (2; 102; 202); an internal surface of said regulation knob (3; 103; 203) and an external surface of said cylindrical collar (30; 130; 230) having respectively locking means and counter-locking means which engage with each other when said regulation knob (3; 103; 203) is in the locked configuration; said regulation knob (3; 103; 203) comprising an internal bush (35; 135; 235) designed to be slidably inserted inside the cylindrical collar (30; 130; 230).

2. Regulator unit (1; 101; 201) according to Claim 1, wherein said locking means and counter-locking means consist of two sets of straight-teeth toothing (31; 131; 231) which are respectively provided on an external circumference of the cylindrical collar (30; 130; 230) and on an internal circumference of the regulation knob (3; 103; 203).

3. Regulator unit (1; 101; 201) according to Claim 2, wherein said cylindrical collar (30; 130; 230) has said toothing (31; 131; 231) on its free end.

4. Regulator unit (1; 101; 201) according to one of the preceding claims, wherein said internal surface of the regulation knob (3; 103; 203) and said external surface of said cylindrical collar (30; 130; 230) have ridges cooperating with each other so as to keep said regulation knob (3; 103; 203) alternately in the locked configuration or in the released configuration.

5. Regulator unit (1; 101; 201) according to Claim 4, wherein said ridges comprise a first radial projection (32; 132; 232) and a second radial projection (33; 133; 233) on one of the two surfaces and a circumferential ring (34; 134; 234) embossed on the other surface; said circumferential ring (34; 134; 234) being constrained between the first radial projection (32; 132; 232) and the second radial projection (33; 133; 233) when the regulation knob (3; 103; 203) is in the released configuration; said circumferential ring (34; 134; 234) being constrained beyond said second radial projection (33; 133; 203) when the regulation knob (3; 103; 203) is in the locked configuration.

6. Regulator unit (1; 101; 201) according to Claim 5, wherein said second radial projection (33; 133; 233) is connected by means of ramps to the surface of the cylindrical collar (30; 130; 320) or the regulation knob (3; 103; 203) so as to allow the linear displacement of the knob (3; 103; 203) between the two - locked and released- configurations.

7. Regulator unit (1; 101; 201) according to one of the preceding claims, wherein said regulation knob (3; 103; 203) in the locked configuration bears against a shoulder (36; 136; 236) of the main body (2; 102; 202).

8. Regulator unit (1; 101; 201) according to one of the preceding claims, wherein said regulation knob (3; 103; 203) has on its external surface a plurality of sectors divided up by a corresponding number of raised ribs (37; 137; 237).

9. Regulator unit (1; 101; 201) according to one of the preceding claims, wherein said regulating valve is a needle valve.

10. Regulator unit (1) according to one of the preceding claims, suitable for being connected to a vacuum source for the suction of fluids inside a safety storage vessel (4) or other container, said regulating valve intercepting a suction flow inside the main body (2) so as to allow regulation of the system suction value.

11. Regulator unit (1) according to Claim 10, wherein said regulation knob (3) is rotationally associated with a cover (52) of the main body (2), said cover being fixed to the remainder of the main body (2) by means of bayonet mount.

12. Regulator unit (101) according to one of the preceding claims, comprising inside the main body (102) a compressed air Venturi aspirator, said regulating valve intercepting the compressed air flow of the Venturi aspirator so as to allow the regulation of the system suction value.

13. Regulator unit (201) according to one of the preceding claims, comprising an inlet designed to be connected to a medical gas distribution source and an outlet for supplying said medical gas to a user apparatus, said regulating valve intercepting a flow supply path between said inlet and said outlet so as to allow regulation of the flowrate of the medical gas supplied at the outlet.

## Patentansprüche

1. Reglereinheit (1; 101; 201) für Fluidabsaugsysteme und/oder Versorgungssysteme für medizinische Zwecke, mit einem Hauptkörper (2; 102; 202), der im Inneren mit wenigstens einem Regelventil versehen ist, wobei das Regelventil eine kontinuierliche Regelung eines Parameters für das Fluidabsaugsystem und/oder für das Versorgungssystem durchführt; und einem Regulierungsknopf (3; 103; 203), der von der Außenseite des Hauptkörpers zugänglich ist (2; 102; 202) und zur Steuerung des Regelventils dient;
wobei der Regulierungsknopf (3; 103; 203) linear entlang einer Achse, wodurch er zwischen einer Regelkonfiguration, bei der der Regulierungsknopf in Bezug auf den Hauptkörper (2; 102; 202) drehbeweglich ist und das Regelventil steuert, und einer Verriegelungskonfiguration, bei der der Regulierungsknopf in Bezug auf den Hauptkörper (2; 102; 202) gegen eine Drehung verriegelt ist, bewegbar ist; wobei der Regulierungsknopf (3; 103; 203) eine schalenförmige Ausgestaltung aufweist,
**dadurch gekennzeichnet, dass** der Regulierungsknopf (3; 103; 203) auf der Oberseite eines zylindrischen Kragens (30; 130; 230), der ein integraler Bestandteil des Hauptkörpers (2; 102; 202) ist, angeordnet ist; und dass eine innere Oberfläche der Regulierungsknopfes (3; 103; 203) und eine Außenfläche des zylindrischen Kragens (30; 130; 230) mit jeweiligen Verriegelungsmitteln und Gegenrastmitteln miteinander in Eingriff stehen, wenn der Regulierungsknopf (3; 103; 203) in der Verriegelungskonfiguration ist; wobei der Regulierungsknopf (3; 103; 203) eine innere Buchse (35; 135; 235) umfasst, die derart ausgebildet ist, um gleitend in dem zylindrischen Kragen (30; 130; 230) eingefügt zu werden.

2. Reglereinheit (1; 101; 201) gemäß Anspruch 1, **dadurch gekennzeichnet, dass** die Verriegelungsmittel und die Gegenrastmittel aus zwei Sätzen von Geradverzahnungen (31; 131; 231) bestehen, die jeweils an einem äußeren Umfang des zylindrischen Kragen (30; 130; 230) und an einem Innenumfang des Regulierungsknopfes (3; 103; 203) angeordnet sind.

3. Reglereinheit (1; 101; 201) gemäß Anspruch 2, **dadurch gekennzeichnet, dass** die Verzahnung (31; 131; 231) an dem freien Ende des zylindrischen Kragens (30; 130; 230) angeordnet ist.

4. Reglereinheit (1; 101; 201) gemäß einem der vorhergehenden Ansprüche, **dadurch gekennzeichnet, dass** die Innenfläche des Regulierungsknopfes (3; 103; 203) und die Außenfläche des zylindrischen Kragens (30; 130; 230) Rippen aufweisen, welche miteinander derart zusammenwirken, so dass der Regulierungsknopf (3; 103; 203) entweder in der Verriegelungskonfiguration oder in der Freigabekonfiguration gehalten wird.

5. Reglereinheit (1; 101; 201) gemäß Anspruch 4, **dadurch gekennzeichnet, dass** die Rippen auf einer der beiden Oberflächen einen ersten radialen Vorsprung (32; 132; 232) und einen zweiten radialen Vorsprung (33; 133; 233) und auf der anderen Oberfläche einen Umfangsring (34; 134; 234) mit Prägung aufweisen, wobei der Umfangsring (34; 134; 234), wenn der Regulierungsknopfes (3; 103; 203) in der Freigabekonfiguration angeordnet ist, zwischen dem ersten radialen Vorsprung (32; 132; 232) und dem zweiten radialen Vorsprung (33; 133; 233) blockiert wird; wobei der Umfangsring (34; 134; 234), wenn der Regulierungsknopf (3; 103; 203) in der Verriegelungskonfiguration angeordnet ist, hinter dem zweiten radialen Vorsprung (33; 133; 203) blockiert ist.

6. Reglereinheit (1; 101; 201) gemäß Anspruch 5, **dadurch gekennzeichnet, dass** der zweite radiale Vorsprung (33; 133; 233) unter Verwendung von Rampen an der Oberfläche des zylindrischen Kragens (30; 130; 320) oder des Regulierungsknopfes (3; 103; 203) befestigt ist, um eine lineare Verschiebung des Knopfes (3; 103; 203) zwischen den beiden Konfigurationen - gesperrt und freigegeben - zu ermöglichen.

7. Reglereinheit (1; 101; 201) nach einem der vorhergehenden Ansprüche, **dadurch gekennzeichnet, dass** der Regulierungsknopf (3; 103; 203) in der Verriegelungskonfiguration gegen eine Schulter (36; 136; 236) des Hauptkörpers (2; 102; 202) drückt.

8. Reglereinheit (1; 101; 201) nach einem der vorhergehenden Ansprüche, **dadurch gekennzeichnet, dass** der Regulierungsknopf (3; 103; 203) auf seiner äußeren Oberfläche eine Vielzahl von Bereichen aufweist, welche durch eine entsprechende Anzahl von erhöhten Rippen (37; 137; 237) unterteilt werden.

9. Reglereinheit (1; 101; 201) nach einem der vorhergehenden Ansprüche, **dadurch gekennzeichnet, dass** das Regelventil ein Nadelventil ist.

10. Reglereinheit (1) nach einem der vorhergehenden Ansprüche, wobei die Reglereinheit dazu geeignet ist mit einer Vakuumquelle zum Ansaugen von Fluiden in das Innere eines Schutzspeicherbehälters (4) oder eines anderen Behälters verbunden zu werden, wobei das Regelventil eine Saugströmung in den Hauptkörper (2) abfängt, um derart die Regulierung des Saugwertes des Systems zu ermöglichen.

11. Reglereinheit (1) gemäß Anspruch 10, **dadurch gekennzeichnet, dass** der Regulierungsknopf (3) drehbar einer Abdeckung (52) des Hauptkörpers (2) zugeordnet ist, wobei die Abdeckung an dem Rest des Hauptkörpers (2) mittels Bajonettverschluss befestigt ist.

12. Reglereinheit (101) nach einem der vorhergehenden Ansprüche, umfassend eine Druckluft-Venturistrahlpumpe im Inneren des Hauptkörpers (102), wobei das Regelventil die Druckluftströmung von der Venturistrahlpurnpe abfängt, um so die Regulierung des Saugwertes des Systems zu ermöglichen.

13. Reglereinheit (201) nach einem der vorhergehenden Ansprüche, umfassend einen Einlass zum Verbinden mit einer medizinischen Gasverteilungsquelle und einem Auslass zum Zufuhren des medizinischen Gases zu einer Benutzervorrichtung, wobei das Regelventil einen Strömungszufuhrweg zwischen dem Einlass und dem Auslass abfängt, wodurch die Regulierung der gelieferten Durchflussmenge des medizinischen Gases am Auslass ermöglicht wird.

## Revendications

1. Unité de régulation (1; 101; 201) pour des systèmes d'aspiration et/ou d'alimentation en fluide à usage médical, comprenant un corps principal (2; 102; 202) muni à l'intérieur d'au moins une valve de régulation qui assure une régulation en continu d'un paramètre dudit système d'aspiration et/ou d'alimentation ; et un bouton de régulation (3; 103; 203) qui est accessible depuis l'extérieur du corps principal (2; 102; 202) et qui est conçu pour contrôler ladite valve de régulation ; ledit bouton de régulation (3; 103; 203) étant mobile de façon linéaire le long d'un axe de celui-ci entre une configuration de régulation, dans laquelle il est mobile en rotation par rapport au corps principal (2; 102; 202) et contrôle la valve de régulation, et une configuration de verrouillage, dans laquelle il est bloqué en rotation par rapport au corps principal (2; 102; 202); ledit bouton de régulation (3; 103; 203) ayant une configuration en forme de tasse, **caractérisée en ce que** ledit bouton de régulation (3 ; 103 ; 203) est monté sur le sommet d'un collet cylindrique (30; 130; 230) qui est d'un seul tenant avec le corps principal (2; 102; 202) ; une surface interne dudit bouton de régulation (3; 103; 203) et une surface externe dudit collet cylindrique (30; 130; 230) comportant respectivement des moyens de verrouillage et des moyens de contre-verrouillage qui viennent en prise ensemble quand ledit bouton de régulation (3; 103; 203) est dans la configuration de verrouillage ; ledit bouton de régulation (3 ; 103 ; 203) comprenant une bague interne (35 ; 135 ; 235) destinée à être insérée de manière coulissante à l'intérieur du collet cylindrique (30 ; 130 ; 230).

2. Unité de régulation (1 ; 101 ; 201) selon la revendication 1, dans laquelle lesdits moyens de verrouillage et moyens de contre-verrouillage se composent de deux jeux de dentures droites (31; 131; 231) qui sont prévus respectivement sur une circonférence externe du collet cylindrique (30; 130; 230) et sur une circonférence interne du bouton de régulation (3; 103; 203).

3. Unité de régulation (1; 101; 201) selon la revendication 2, dans laquelle ledit collet cylindrique (30; 130; 230) porte ladite denture (31; 131; 231) sur son extrémité libre.

4. Unité de régulation (1; 101; 201) selon l'une des revendications précédente, dans laquelle ladite surface interne du bouton de régulation (3; 103; 203) et ladite surface externe dudit collet cylindrique (30; 130; 230) possèdent des nervures coopérant entre elles de sorte à maintenir ledit bouton de régulation (3; 103; 203) soit dans la configuration de verrouillage soit dans la configuration relâchée.

5. Unité de régulation (1; 101; 201) selon la revendication 4, dans laquelle lesdites nervures comprennent une première projection radiale (32; 132; 232) et une seconde projection radiale (33; 133; 233) sur l'une des deux surfaces et un anneau circonférentiel (34; 134; 234) en relief sur l'autre surface, ledit anneau circonférentiel (34; 134; 234) étant contraint entre la première projection radiale (32; 132; 232) et la deuxième projection radiale (33; 133; 233) lorsque le bouton de régulation (3; 103; 203) est dans la configuration relâchée ; ledit anneau circonférentiel (34; 134; 234) étant contraint au-delà de ladite seconde projection radiale (33; 133; 203) quand le bouton de régulation (3; 103; 203) est dans la configuration verrouillée.

6. Unité de régulation (1; 101; 201) selon la revendication 5, dans laquelle ladite seconde projection radiale (33; 133; 233) est reliée au moyen de rampes à la surface du collet cylindrique (30; 130; 320) ou au bouton de régulation (3; 103; 203) de manière à permettre le déplacement linéaire du bouton (3; 103; 203) entre les deux configurations-verrouillée et relâchée.

7. Unité de régulation (1; 101; 201) selon l'une des revendications précédentes, dans laquelle ledit bouton de régulation (3; 103; 203), dans la configuration verrouillée, bute contre un épaulement (36; 136; 236) du corps principal (2; 102; 202).

8. Unité de régulation (1 ; 101 ; 201) selon l'une des revendications précédentes, dans laquelle ledit bouton de régulation (3; 103; 203) présente sur sa surface extérieure une pluralité de secteurs divisés par un nombre correspondant de nervures en relief (37; 137 ; 237).

9. Unité de régulation (1 ; 101 ; 201) selon l'une des revendications précédentes, dans laquelle ladite valve de régulation est une valve à aiguille.

10. Unité de régulation (1) selon l'une des revendications précédentes, laquelle est apte à être reliée à une source de vide pour l'aspiration de fluides à l'intérieur d'un récipient de stockage de sécurité (4) ou d'un autre récipient, ladite valve de régulation interceptant un flux d'aspiration à l'intérieur du corps principal (2) de manière à permettre la régulation de la valeur d'aspiration du système.

11. Unité de régulation (1) selon la revendication 10, dans laquelle ledit bouton de régulation (3) est associé en rotation à un couvercle (52) du corps principal (2), ledit couvercle étant fixé sur le reste du corps principal (2) au moyen d'un système à baïonnette.

12. Unité de régulation (101) selon l'une des revendications précédentes, laquelle comprend à l'intérieur du corps principal (102) un venturi d'aspiration à air comprimé, ladite valve de régulation interceptant le flux d'air comprimé du venturi d'aspiration de manière à permettre la régulation de la valeur d'aspiration du système.

13. Unité de régulation (201) selon l'une des revendications précédentes, laquelle comprend une entrée conçue pour être reliée à une source de distribution de gaz médical et une sortie pour fournir ledit gaz à usage médical à un dispositif utilisateur, ladite valve de régulation interceptant un chemin de flux d'alimentation entre ladite entrée et ladite sortie de manière à permettre la régulation du débit du gaz médical fourni à la sortie.
